Europäisches Patentamt

European Patent Office

Office européen des brevets

(19)

(11) Publication number: **0 142 597**
**B1**

(12) **EUROPEAN PATENT SPECIFICATION**

(45) Date of publication of patent specification: **01.02.89**

(51) Int. Cl.⁴: **A 61 K 7/50**

(21) Application number: **84102855.8**

(22) Date of filing: **15.03.84**

(54) **Use of a mixture of compounds for the manufacture of a composition for the treatment of Buerger's disease.**

(30) Priority: **22.11.83 JP 220246/83**

(43) Date of publication of application:
**29.05.85 Bulletin 85/22**

(45) Publication of the grant of the patent:
**01.02.89 Bulletin 89/05**

(84) Designated Contracting States:
**AT CH DE FR GB IT LI SE**

(56) References cited:
**FR-A- 722 490**

**HAGERS HANDBUCH DER PHARMAZEUTISCHEN PRAXIS, vol. VIIA, no. 4, 1971, pp. 266-268, Springer-Verlag, BERLIN (DE)**

(73) Proprietor: **Kao Corporation
14-10, Nihonbashi Kayabacho 1-chome
Chuo-Ku Tokyo 103 (JP)**

(72) Inventor: **Yorozu, Hidenori
1729, Mashiko Mashikomachi
Haga-gun Tochigi-ken (JP)**
Inventor: **Eguchi, Yasuteru
513-6, Minemachi
Utsunomiya-shi Tochigi-ken (JP)**

(74) Representative: **Wächtershäuser, Günter, Dr.
Tal 29
D-8000 München 2 (DE)**

EP 0 142 597 B1

Courier Press, Leamington Spa, England.

## Description

### i) Field of the Invention:

This invention relates to curative composition for Buerger's disease and more particularly, to external curatives therefor.

### ii) Buerger's Disease:

Buerger's disease is also called thromboangiitis obliterans and is a disease found primarily in young and middle-aged males, causing segmental lesions of the arteries and veins of the limbs and, rarely, of the internal organs. The disease is characterized by diffuse, inflammatory, proliferous and non-suppurative changes of the entire layer of the affected blood vessels and thrombatic obstruction thereof. The total number of the patients suffering from Buerger's disease in Japan is estimated at between 4000 and 5000. In general, intermittent claudication, rest pain, and spontaneous gangrene are considered to be the three major symptoms of the disease. Depending on the degree of peripheral ischemia, peculiar paraesthesia and pains such as paralysis, rest pain and intermittent claudication bring about, leading to occurrence of atrophy and sclerosis. The skin assumes luster, the sweat-gland withers, and the horniness increases, with the result that cutaneous horn, collosity and pachydermia may often be formed on peripheries of the limbs, with nails being observed to be undergrown. Moreover, when affected with Buerger's disease, one will receive a wound even by a slight degree of impetus which would be usually negligible and present a condition called spontaneous gangrene.

It is usually accepted that for cure of the intermittent claudication which is a skeletal muscle ischemia at the time of a movement, a blood circulation reconstruction technique is the most suitable but a medical therapy is not effective. The blood circulation reconstruction technique is considered to be the most effective means for the rest pain and gangrene on leg ends which result from the shortage in amount of blood flow upon rest. However, one has to rely on a medical therapy in the absence of operational adaptation.

### iii) Description of the Prior Art:

For the medical therapy, there are used vasodilators, anticoagulants, plastocyte coagulation inhibitors, low molecular weight dextran, erythrocyte deformation accelerators, and defibrination drugs. Among them vasodilators have been widely used up to now. However, when they are generally dosed, normal blood vessels are dilated a phenomenon of blood flow takes place, and a systematic blood pressure is lowered. Thus, the efficacy of vasodilators is considered to be doubtful. The most important problem resides in the fact that peripheral blood vessels remote from occluded portions of arteries which are ischemic have been already relaxed, so that even though a vasodilator is dosed, there is little possibility of further dilating the vessels. In addition, no drugs are known for selectively dilating collateral vessels.

In recent years, continuous intraarterial injection of prostagladin $E_1$ is effected with successful results. However, the operation of continuously injecting a medical fluid while keeping a catheter in an artery involves a degree of danger, imposing a great burden on the part of patient. Additionally, there are not a few adiaphorous cases.

From Hagers Handbuch der Pharmazeutischen Praxis, vol. VII A, No. 4, 1971, pages 266 to 268, and from FR—A—722 490, compositions comprising at least one carbonate and at least one acidic substance are known which are used as bath additives.

### Summary of the Invention

In view of the above, we have intensively studied curative compositions for Buerger's disease and found that carbon dioxide dissolved in water which is obtained from a composition comprising carbonates and acidic substances is effective as a curative means for Buerger's disease.

Accordingly, the present invention is the use of of a composition comprising at least one carbonate and at least one acidic substance, whereby 0.01 wt.-% of said composition dissolved in water provides an aqueous solution having a pH of 4 to 7 for the manufacture of an external curative for the treatment of Buerger's disease.

### Detailed Description of the Invention and Preferred Embodiments

The carbonates useful in the present invention include, for example, sodium hydrogencarbonate, potassium hydrogencarbonate, sodium carbonate, potassium carbonate, and sodium sesquicarbonate. These carbonates may be used singly or in combination.

The acidic substances include, for example, organic acids such as citric acid, tartaric acid, malic acid, malonic acid, succinic acid, and fumaric acid and phosphoric acid. Salts of these acids may also be used including sodium citrate, sodium succinate and sodium phosphate.

When the curative composition of the invention is dissolved in water, carbon dioxide is generated. Under alkaline conditions, the carbon dioxide exists as carbonate or bicarbonate ions, so that no blood flow accelerating effect is recognized. In contrast, it exists as carbon dioxide molecules under acidic conditions, thus showing the blood flow accelerating effect. Accordingly, the amounts of the acidic substances and the carbonates are determined so that an aqueous solution of 0.01 wt% of the curative composition has a pH of from 4 to 7. The pH lower than 4 is unfavorable because such a solution tends to wound.

The amount of the acidic substance which satisfies the pH requirement may vary depending on the type of substance. For instance, with succinic acid, it is conveniently used in an amount of 20—200 wt% of carbonate (calculated as sodium hydrogencarbonate) and with sodium succinate, its amount is in the range of from 40 to 400 wt% of carbonate (calculated as sodium hydrogencarbonate). The curative composition of the invention is preferably applied to the affected part in the form of an aqueous solution or hot solution dissolving the composition therein.

Alternatively, one may take a bath after dissolution of the composition in the bath.

The curative composition may be used in an amount of 5 to 250 g by dissolution in 10—200 liters of water or hot water. It is preferred that the solution is applied to the affected part several times a day for 10—30 minutes for each application. This is continued over several days to several months by which development of the symptons is stopped or the symptoms are improved.

The curative composition of the invention is externally applied to the affected part, and the acidic substances, carbonates and carbon dioxide generated therefrom are harmless against the skin.

The curative composition of the invention may further comprise, aside from the essential acidic substances and carbonates, antiphlogistics, germicides, wound cure promoters, vitamins, perfumes and colorants. The curative composition may be prepared in the form of powder, granules, and tablets. For the preparation, excipients, binders, disintegrators, and lubricants may be added, if necessary.

As will be seen from the foregoing, the curative composition for Buerger's disease according to the invention can remarkably improve the symptoms of the disease when externally applied to the affected part. The composition can be applied very simply and has very high safety because it may be applied externally.

The present invention is described more particularly by way of examples.

### Example 1

A forty six years old male was diagnosed as having thromboangiitis obliterans (Buerger's disease). Three years after the diagnosis, an ischemic ulcer was formed on the right first toe and was hard to cure. The ulcer extended below the nail, which was subsequently removed to effect the conservative treatment. Although prostaglandin $E_1$ was dosed for about 1 month, the curative tendency was not good. Accordingly, the right first toe was cut off along with the left second toe whose ulcer exacerbated.

About one month after the amputation, 50 g of a curative composition having a formulation of Example 3 was dissolved in 150 liters of water to give an aqueous solution. Both legs were immersed in the aqueous solution by full bath at a temperature of 40°C for 10 minutes. Several days after commencement of the immersion, an improvement in rest pain was observed, making the use of an analgesic unnecessary. When the use of the curative composition of the invention was interrupted (3 days), the granulation tissue was observed to exacerbate with reappearance of rest pain. The curative composition was again used. In 3 days, the wound was much changed for the better, with an improvement of the granulation tissue and a disappearance of rest pain. Thereafter, the treatment was continued for about 1 month, by which the right first toe was cured. No pain was felt at the cut end of the left second toe.

Throughout the above treatment process, prostagladin $E_1$ preparation was used in combination and the curative composition of the invention showed a significant improvement of the symptoms.

### Example 2

A 59 years old male was found with an ischemic intractable ulcer at the right first toe thereof, extended to below the nail. The patient was presumed to suffer from the thromboangiitis obliterans (Buerger's disease). After removal of the nail, a curative tendency was observed by the use of a combination of prostaglandin and panaldin. However, use of a curative composition of the invention was started in order to expect an early cure of the wound. About two months after the removal of the nail, the affected part was immersed, twice a day at 40°C for 10 minutes, in an aqueous solutiuon of 50 g of a curative composition having a composition of Example 3 in 150 liters of water. In 4 months after commencement of the immersion, the wound was cured while leaving redness owing to the insufficiency of cutization.

### Example 3

| | | | |
|---|---|---|---|
| Sodium bicarbonate | 44 | (parts by weight) |
| Succinic acid | 38 | |
| Excipient | 18 | |
| | 100 | |

### Example 4

| | | | |
|---|---|---|---|
| Sodium bicarbonate | 50 | (parts by weight) |
| Citric acid | 30 | |
| Sodium sulfate | 10 | |
| Excipient | 10 | |
| | 100 | |

### Example 5

| | | | |
|---|---|---|---|
| Sodium bicarbonate | 40 | (parts by weight) |
| Fumaric acid | 25 | |
| Sodium polyacrylate | 3 | |
| Excipient | 32 | |
| | 100 | |

**Claim**

Use of a composition comprising at least one carbonate and at least one acidic substance, whereby 0.01 wt.-% of said composition dissolved in water provides an aqueous solution having a pH of 4 to 7 for the manufacture of an external curative for the treatment of Buerger's disease.

**Patentanspruch**

Verwendung einer Zusammensetzung, umfassend mindestens ein Carbonat und mindestens eine saure Substanz, wobei 0,01 Gew.-% der Zusammensetzung, aufgelöst in Wasser, eine wäßrige Lösung mit einem pH von 4 bis 7 schaffen, zur Herstellung eines äußerlich anzuwendenden Heilmittels für die Behandlung der Buergerschen Krankheit (Buerger's disease).

**Revendications**

Utilisation d'une composition comprenant au moins un carbonate et au moins une substance acide, 0,01% en poids de ladite composition dissous dans l'eau donnant une solution aqueuse ayant un pH allant de 4 à 7, pour la fabrication d'un médicament pour le traitement par voie externe de la maladie de Buerger.